# EUROPEAN PATENT SPECIFICATION

(11) **EP 3 135 278 B1**
(45) Date of publication and mention of the grant of the patent: **12.06.2019**
(21) Application number: 15193428.8
(22) Date of filing: 06.11.2015
(51) Int. Cl.: A61K 31/18, A61K 36/48, A61K 36/481, A61P 35/00, A61P 13/08

(54) **BENIGN PROSTATIC HYPERPLASIA ADD-ON THERAPY**
ZUSÄTZLICHE THERAPIE VON GUTARTIGER PROSTATAHYPERPLASIE
THÉRAPIE COMPLÉMENTAIRE DE L'HYPERPLASIE BÉNIGNE DE LA PROSTATE

(30) Priority: 18.08.2015 US 201514828522
(43) Date of publication of application: 01.03.2017
(73) Proprietor: Golden Biotechnology Corporation, Jersey City NJ 07302 (US)
(72) Inventor: LIU, Sheng-Yung, New Taipei City Taiwan Danshuei (CN)
(74) Representative: Wichmann, Hendrik

(56) References cited:
- EP-A1- 1 808 178
- US-B2- 7 749 546
- Anonymous: "NCT02313233 on 2015_08_05: ClinicalTrials.gov Archive", , 5 August 2015 (2015-08-05), XP055302958, Retrieved from the Internet: URL:https://clinicaltrials.gov/archive/NCT 02313233/2015_08_05 [retrieved on 2016-09-15]
- Anonymous: "Golden Biotechnology Corp | uMooze", , 4 July 2015 (2015-07-04), XP055303059, Retrieved from the Internet: URL:https://web.archive.org/web/2015070421 5154/http://www.goldenbiotech.com.tw/en/pr oduct4.html [retrieved on 2016-09-15]
- Anonymous: "Various treatment options for benign prostatic hyperplasia: A current update", , 1 January 2012 (2012-01-01), XP055303162, Retrieved from the Internet: URL:http://www.ncbi.nlm.nih.gov/pmc/articl es/PMC3425142/?report=printable [retrieved on 2016-09-16]
- Anonymous: "Benign prostatic hyperplasia: An overview of existing treatment", , 1 January 2011 (2011-01-01), XP055303165, Retrieved from the Internet: URL:http://www.ncbi.nlm.nih.gov/pmc/articl es/PMC3062123/?report=printable [retrieved on 2016-09-16]

## Description

### BACKGROUND OF THE INVENTION

Benign prostatic hyperplasia (BPH), also called benign enlargement of the prostate (BEP or BPE), adenofibromyomatous hyperplasia and benign prostatic hypertrophy, is a benign increase in size of the prostate.

BPH involves hyperplasia of prostatic stromal and epithelial cells, resulting in the formation of large, fairly discrete nodules in the transition zone of the prostate. When sufficiently large, the nodules impinge on the urethra and increase resistance to flow of urine from the bladder. This is commonly referred to as "obstruction," although the urethral lumen is no less patent, only compressed. Resistance to urine flow requires the bladder to work harder during voiding, possibly leading to progressive hypertrophy, instability, or weakness (atony) of the bladder muscle. Although prostate specific antigenlevels may be elevated in these patients because of increased organ volume and inflammation due to urinary tract infections, BPH does not lead to cancer or increase the risk of cancer.

Benign prostatic hyperplasia (BPH) is commonly found in aging men and is characterized by the presence of stromal and epithelial cell hyperplasia beginning in the periurethral zone of the prostate. BPH is prevalent in 25% of those above 55 years of age. It usually reaches 50-73% by the age of 90 years. The major clinical effect of BPH is constriction of the urethra, which prevents complete voiding of the bladder. However, the irritative symptoms of BPH (urinary urgency, urinary frequency, and nocturia) have a greater impact on quality of life than do the obstructive symptoms (hesitancy, straining, decreased urinary flow rate, urinary retention, and postvoid dribbling). The patients can option for watchful waiting, behavior modification, surgery, medical therapy or nutritional therapies and supplements.

The two main medications for management of BPH are alpha blockers and 5α-reductase inhibitors (reviewed in Shrivastava & Gupta, J Midlife Health 3(1): 10-19, 2012; and Dhingra & Bhagw at, Indian J Pharmacol. 43(1): 6-12, 2011). Herbal remedies are a commonly used treatment, and several are approved in European countries and available in the USA. Saw palmetto extract from *Serenoa repens* is one of the most commonly used and studied, having showed some promise in early studies. US7749546 discloses a composition of Astragalux radix extract and soy isoflavones in a ratio of 1:2, 2:1 or 5:1, respectively. EP1808178 discloses a composition comprising Astragalus radix extracts for treating prostate cancer and suppressing prostatic hyperplasia. 'uMooze' is marketed by Golden Biotechnology Corp. (XP055303059), and clinical trial NCT02313233 describes an outline for a study to research the possible benefit of Umooze as add-on therapy in benign prostatic hyperplasia.

### SUMMARY OF THE INVENTION

In one aspect provided herein are compositions suitable for use in the treatment of benign prostatic hyperplasia (BPH) in a subject comprising an extract or powder of a herbal mixture comprising *Astragalus membranaceus* and *Glycine max,* and an anti-BPH agent, as defined in the claims. In certain aspect, said *Astragalus membranaceus* comprises at least one, two, three or four components selected from the group consisting of polysaccharides such as APS-1 and/or APS-II, or the like; triterpenoid saponins such as acetylastragaloside I, astragaloside I, astragaloside II, astragaloside IV, or the like; flavonoids (e.g., quercetin), amino acids such as asparagines; the trace elements of Fe, Mn,Se, Zn, etc. In certain aspect, said *Glycine max* comprises genistein, daidzein, or the like, or a combination thereof.

In another aspect provided herein is a composition for use in the treatment of benign prostatic hyperplasia (BPH) in a subject comprising an extract or powder of a herbal mixture comprising *Astragalus membranaceus* and *Glycine max* as add-on therapy, with an anti-BPH agent, as further defined in the claims. In certain aspect, said *Astragalus membranaceus* comprises at least one, two, three or four components selected from the group consisting of polysaccharides such as APS-1 and/or APS-II; triterpenoid saponins such as acetylastragaloside I, astragaloside I, astragaloside II, astragaloside IV, or the like; flavonoids (e.g., quercetin); and amino acids such as asparagines. In further aspect, said *Glycine max* comprises genistein, daidzein, or the like, or combination thereof.

### BRIEF DESCRIPTION OF THE DRAWINGS

The features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

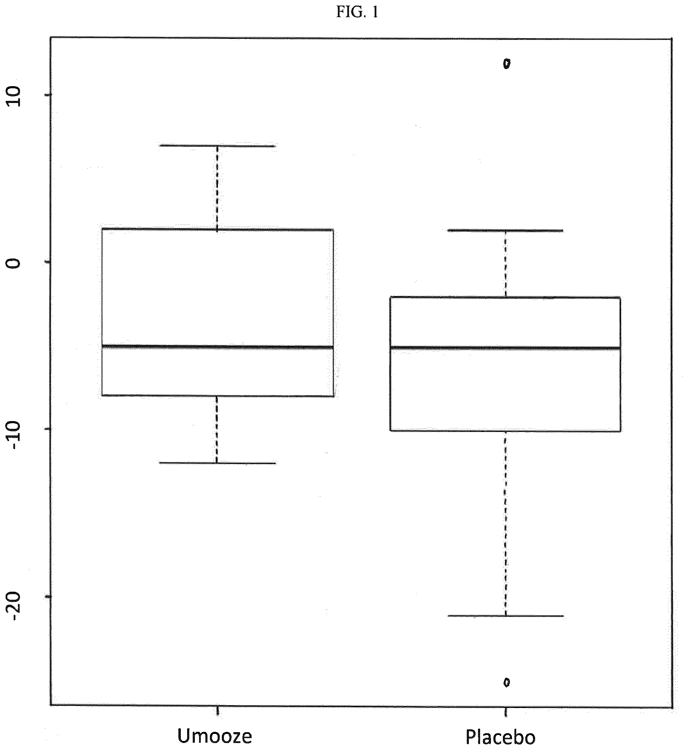

FIG. 1 illustrates an exemplary Box plot of IPSS for the difference between V1 and V6, Upper bond (the upper solid line), Q3 (top of box), median (Bold solid line), Q1 (bottom of box) and Lower bond (the lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

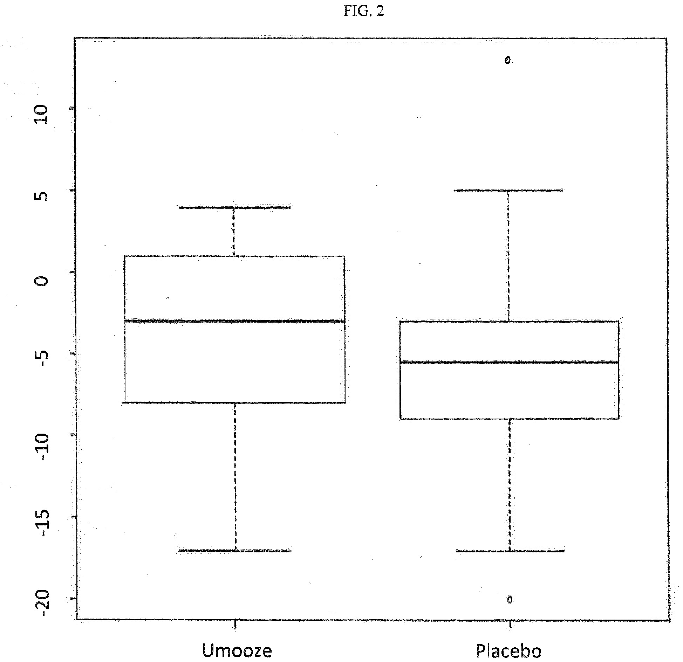

FIG. 2 illustrates an exemplary Box plot of IPSS for the difference between V1 and V5, Upper bond (the upper solid line), Q3 (top of box), median (Bold solid line), Q1 (bottom of box) and Lower bond (the lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

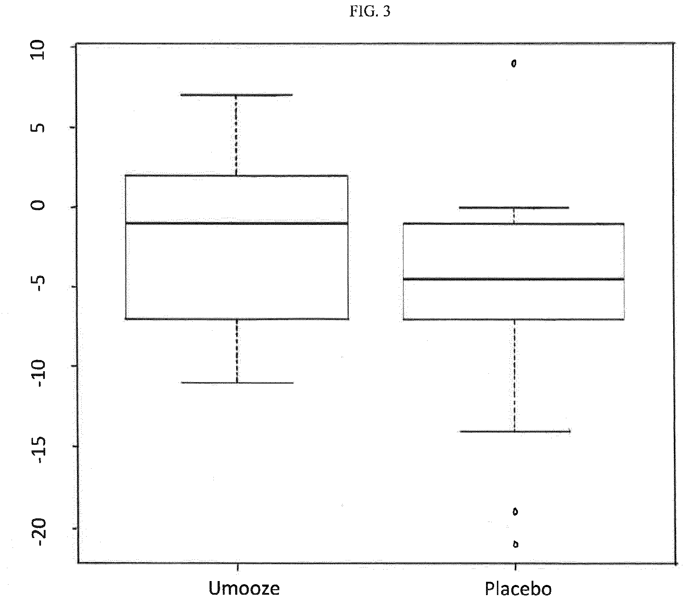

FIG. 3 illustrates an exemplary Box plot of IPSS for the difference between V1 and V4, Upper bond (The upper solid line), Q3 (Top of box), median (Bold solid line), Q1 (Bottom of box) and Lower bond (The lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

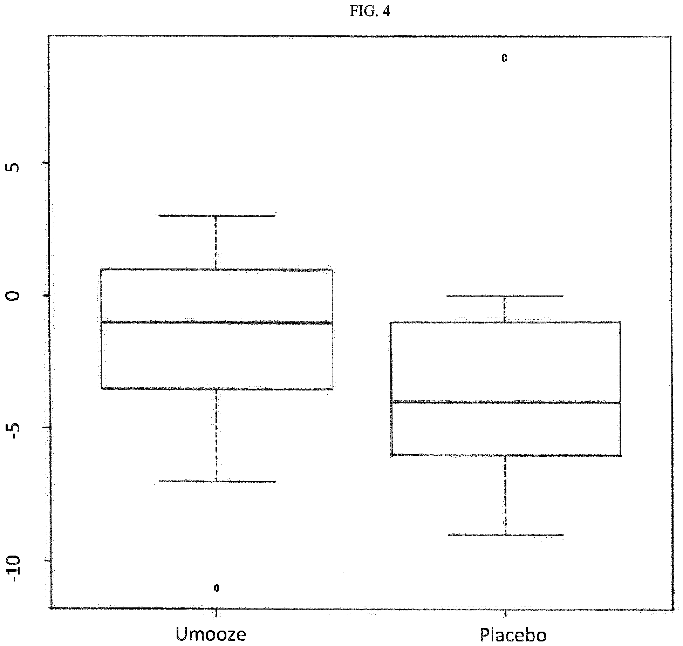

FIG. 4 illustrates an exemplary Box plot of IPSS for the difference between V1 and V6 in Harnalidge® 0.4 mg once daily treatment subjects, Upper bond (The upper solid line), Q3 (Top of box), median (Bold solid line), Q1 (Bottom of box) and Lower bond (The lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

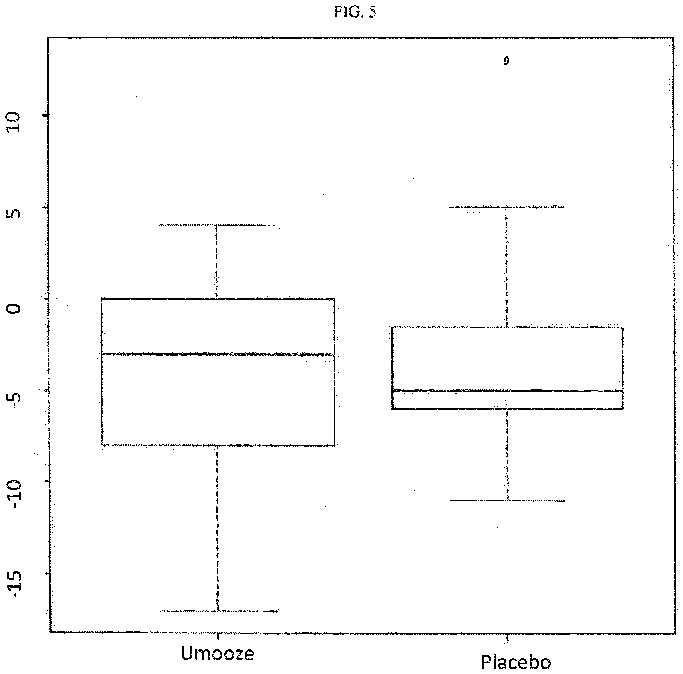

FIG. 5 illustrates an exemplary Box plot of IPSS for the difference between V1 and V5 in Harnalidge® 0.4 mg once daily treatment subjects, Upper bond (The upper solid line), Q3 (Top of box), median (Bold solid line), Q1 (Bottom of box) and Lower bond (The lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

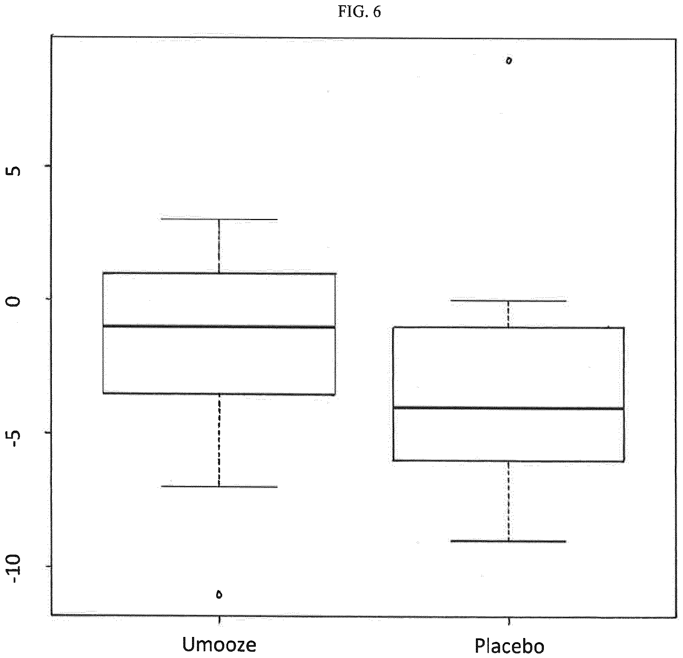

FIG. 6 illustrates an exemplary Box plot of IPSS for the difference between V1 and V4 in Harnalidge® 0.4 mg once daily treatment subjects, Upper bond (The upper solid line), Q3 (Top of box), median (Bold solid line), Q1 (Bottom of box) and Lower bond (The lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

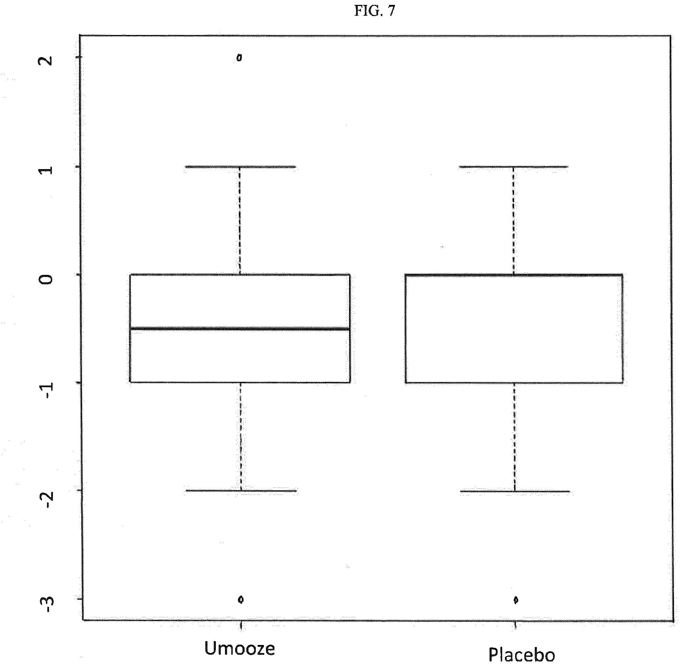

FIG. 7 illustrates an exemplary Box plot of QoL for the difference between V1 and V6, Upper bond (the upper solid line), Q3 (top of box), median (Bold solid line), Q1 (bottom of box) and Lower bond (the lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

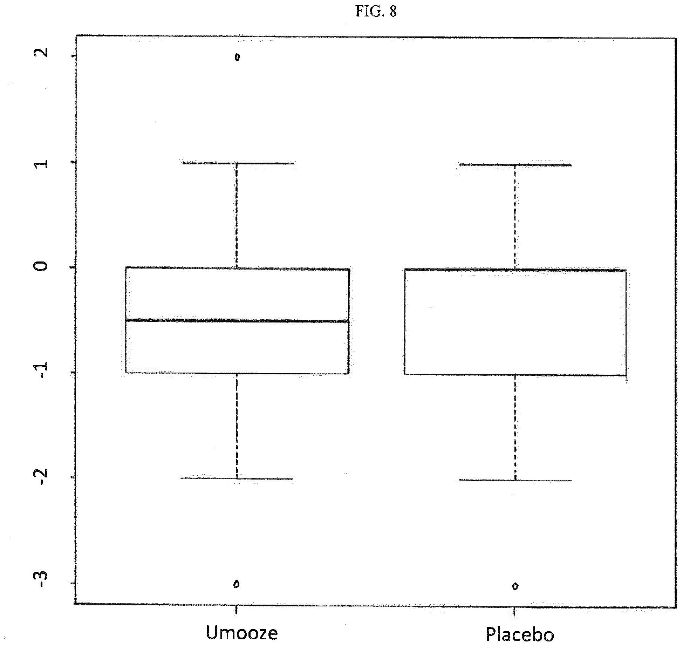

FIG. 8 illustrates an exemplary Box plot of QoL for the difference between V1 and V5, Upper bond (the upper solid line), Q3 (top of box), median (Bold solid line), Q1 (bottom of box) and Lower bond (the lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

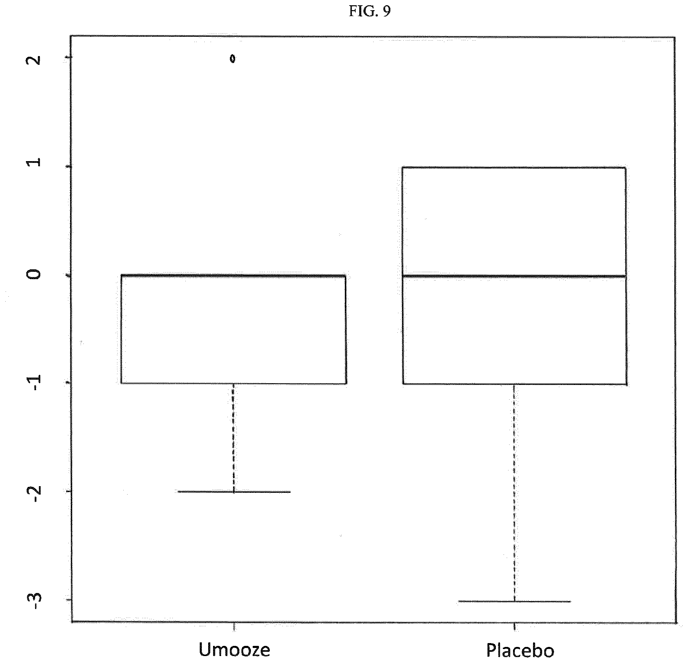

FIG. 9 illustrates an exemplary Box plot of QoL for the difference between V1 and V4, Q3 (top of box), median (Bold solid line), Q1 (bottom of box) and Lower bond (the lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

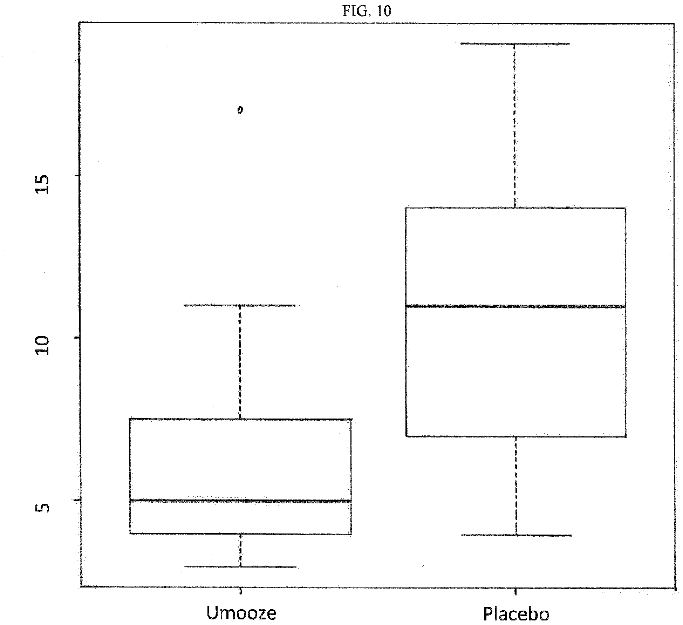

FIG. 10 illustrates an exemplary Box plot of QoL for the difference between V1 and V6 in Harnalidge® 0.4 mg once daily treatment subjects, Q3 (Top of box), median (Bold solid line), Q1 (Bottom of box) and Lower bond (The lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

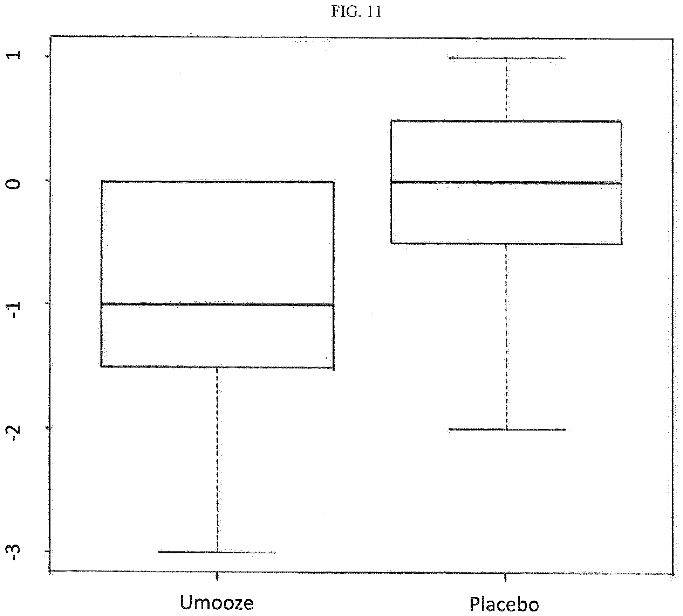

FIG. 11 illustrates an exemplary Box plot of QoL for the difference between V1 and V5 in Harnalidge® 0.4 mg once daily treatment subjects, Upper bond (The upper solid line), Q3 (Top of box), median (Bold solid line), Q1 (Bottom of box) and Lower bond (The lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

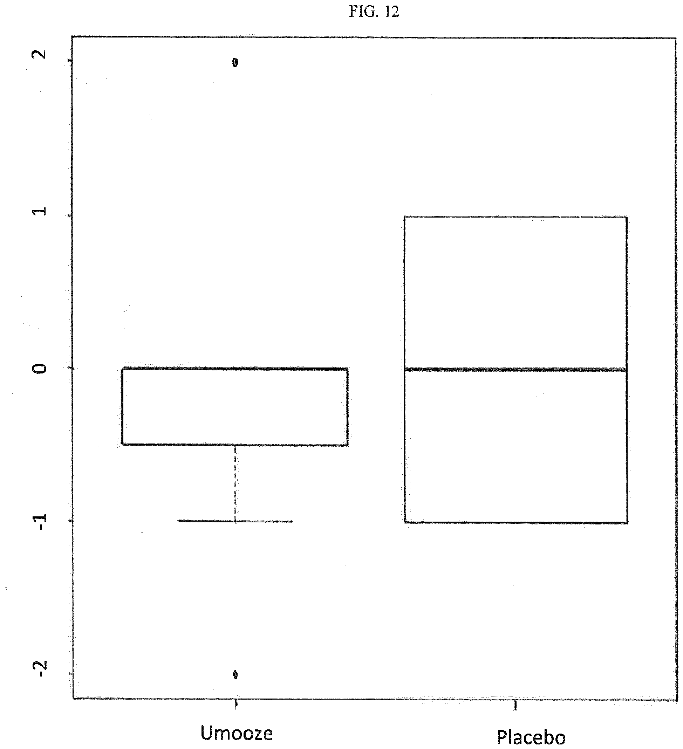

FIG. 12 illustrates an exemplary Box plot of QoL for the difference between V1 and V4 in Harnalidge® 0.4 mg once daily treatment subjects, Q3 (Top of box), median (Bold solid line), Q1 (Bottom of box) and Lower bond (The lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

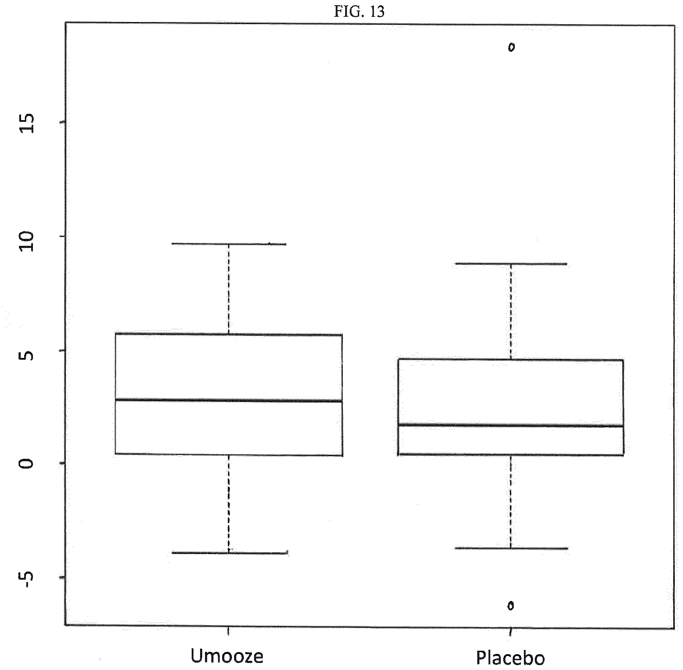

FIG. 13 illustrates an exemplary Box plot of Qmax for the difference between V1 and V6, Upper bond (the upper solid line), Q3 (top of box), median (Bold solid line), Q1 (bottom of box) and Lower bond (the lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

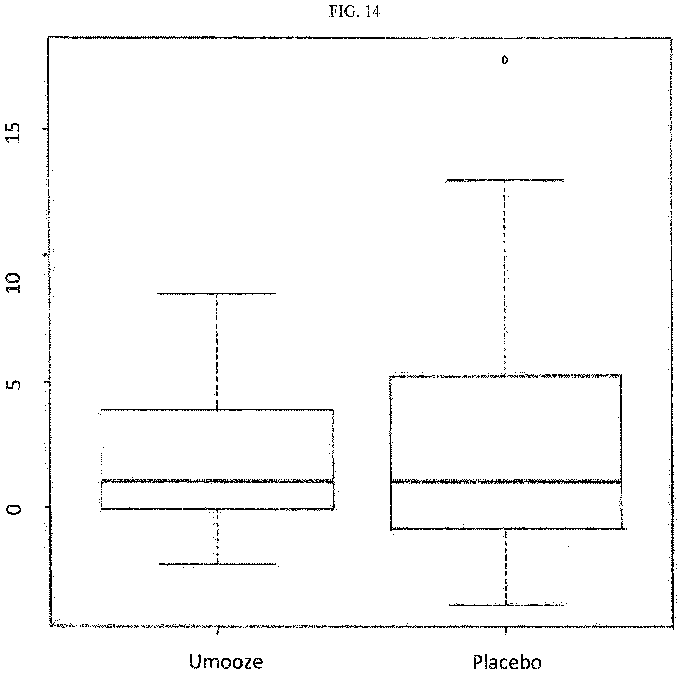

FIG. 14 illustrates an exemplary Box plot of Qmax for the difference between V1 and V4, Upper bond (the upper solid line), Q3 (top of box), median (Bold solid line), Q1 (bottom of box) and Lower bond (the lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

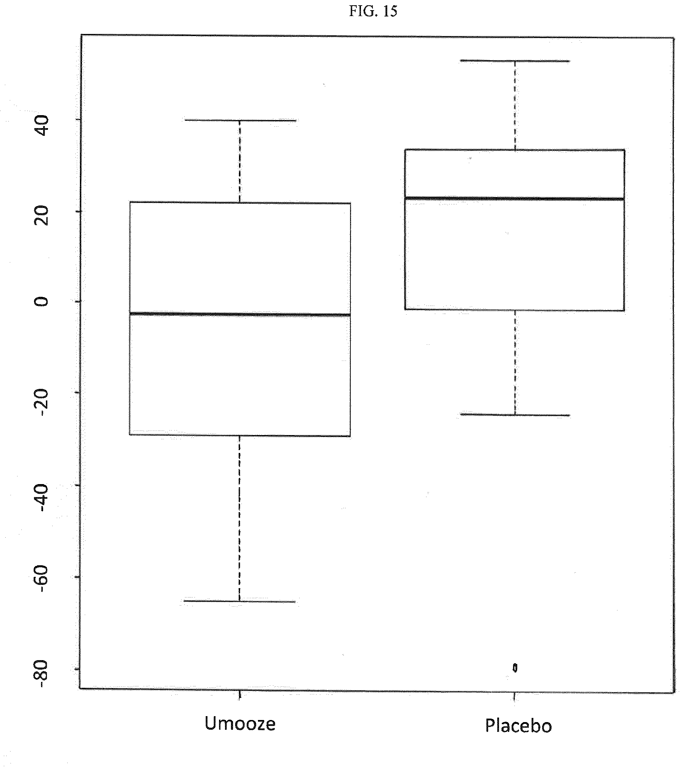

FIG. 15 illustrates an exemplary Box plot of PVR for the difference between V1 and V6, Upper bond (the upper solid line), Q3 (top of box), median (Bold solid line), Q1 (bottom of box) and Lower bond (the lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

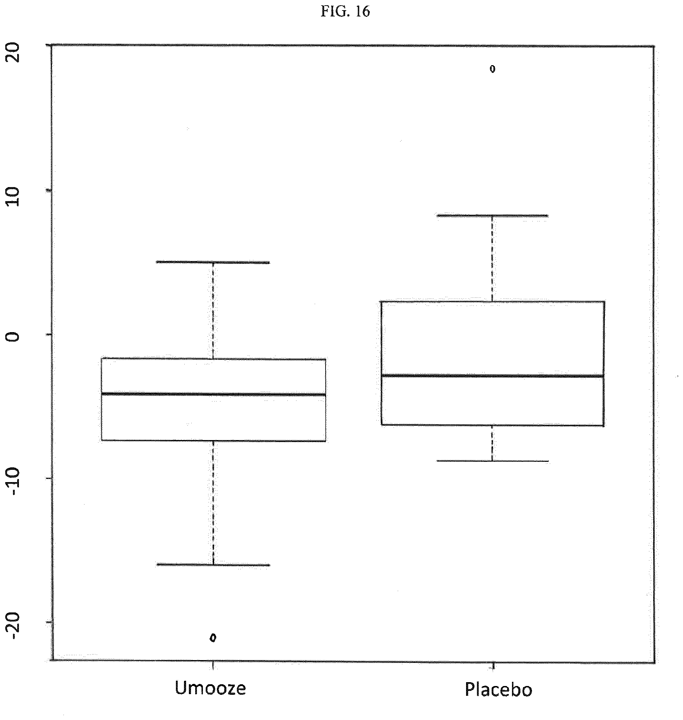

FIG. 16 illustrates an exemplary Box plot of prostate volume for the difference between V1 and V6, Upper bond (the upper solid line), Q3 (top of box), median (Bold solid line), Q1 (bottom of box) and Lower bond (the lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

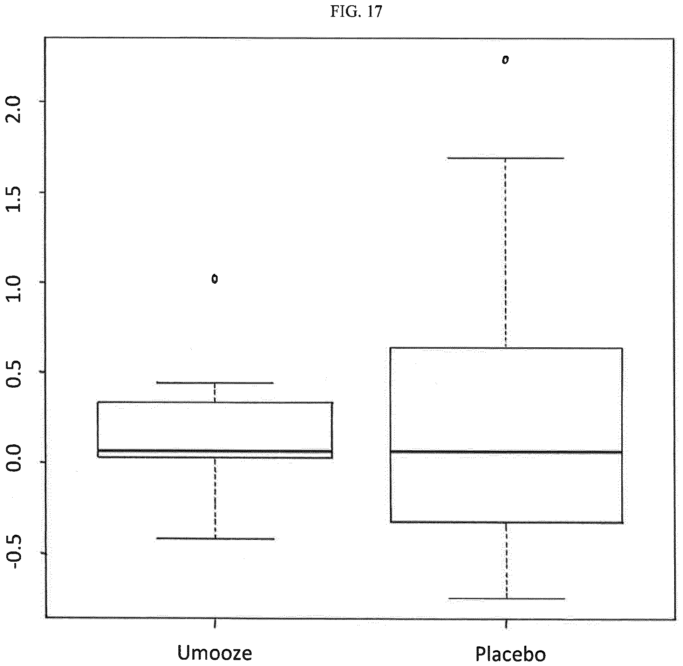

FIG. 17 illustrates an exemplary Box plot of PSA for the difference between V1 and V6, Upper bond (the upper solid line), Q3 (top of box), median (Bold solid line), Q1 (bottom of box) and Lower bond (the lower solid line). Q1: the first quartile; Q3 the third quartile; Define Interquartile range (IQR= Q3-Q1); Upper bond= Q3+1.5 IQR; Lower bond= Q1-1.5 IQR; Non-full circle: Outlier.

### DETAILED DESCRIPTION OF THE INVENTION

Benign prostatic hyperplasia (BPH), also called benign enlargement of the prostate (BEP or BPE), adenofibromyomatous hyperplasia and benign prostatic hypertrophy (technically incorrect usage), is a benign increase in size of the prostate. Benign prostatic hyperplasia symptoms are classified as storage or voiding. Storage symptoms include urinary frequency, urgency (compelling need to void that cannot be deferred), urgency incontinence, and voiding at night (nocturia). Urinary incontinence may occur, and nocturia may contribute to insomnia. Voiding symptoms include urinary hesitancy (difficulty initiating the stream), straining to void, weak or intermittent stream (starts and stops), and incomplete bladder emptying. Pain and dysuria are usually not present. These storage and voiding symptoms are evaluated using the International Prostate Symptom Score (IPSS) questionnaire, designed to assess the severity of BPH. Nocturia is a condition in which a subject wakes up during the night because the subject has to urinate.

The two main medications for management of BPH are alpha blockers and 5α-reductase inhibitors. Alpha blockers are the most common choice for initial therapy. They include for example doxazosin, terazosin, alfuzosin, tamsulosin, silodosin, and the like. They have a small to moderate benefit. All five are equally effective but have slightly different side effect profiles. The older drugs phenoxybenzamine and prazosin are less recommended. Alpha blockers relax smooth muscle in the prostate and the bladder neck, thus decreasing the blockage of urine flow. Common side effects of alpha blockers include orthostatic hypotension, (a head rush or dizzy spell when standing up or stretching), ejaculation changes, headaches, nasal congestion, and weakness. Non-selective alpha blockers such as terazosin and doxazosin may also require titration as they can cause syncope if the dose is too high. Side effects can also include erectile dysfunction.

The 5α-reductase inhibitors finasteride and dutasteride are another treatment option. These medications inhibit 5α-reductase, which in turn inhibits production of DHT, a hormone responsible for enlarging the prostate. Effects may take longer to appear than alpha blockers, but they persist for many years. When used together with alpha blockers, a reduction of BPH progression to acute urinary retention and surgery has been noted in patients with larger prostates. Side effects include decreased libido and ejaculatory or erectile dysfunction. Antimuscarinics such as tolterodine may also be used, especially in combination with alpha blockers. They act by decreasing acetylcholine effects on the smooth muscle of the bladder, thus helping control symptoms of an overactive bladder. Sildenafil citrate and Tadalafil are among possible medicines to use for BPH treatments.

Besides the small to moderate benefit for treating BPH, all of the exemplary medicines exist a small to moderate side effects as well.

Recently, attention has been paid to Traditional Chinese Medicines (TCM), especially their potential as add-on therapy.

Add-on therapy or adjuvant therapy (also called adjunct therapy or adjunctive therapy or care), is therapy that is given in addition to the primary, main, or initial therapy to maximize its effectiveness. As an adjuvant agent (add-on agent) modifies the effect of another agent, so add-on therapy modifies other therapy.

For example, Huangqi, (such as *astragalus* radix (AR)) is the dried root of *Astragalus membranaceus Bge. Var*. *mongholicus* and is used as a tonic in the traditional Chinese medicine. It has been used extensively as an adjuvant in cancer treatment and as a phytochemical immune modulator. Based on the relevant researches in Mainland China during recent years, the AR has various effects such as: to enhance the immunity function, improve the activity of killer cells, enhance the resistance to diseases, anti-aging, anti-oxidation and nutritional anemia treatment, improve the cardiovascular system, multiple virus resistance and anti-cancer etc. *Astragalus* (syn. *Astragale, Astragale à Feuilles de Réglisse, Astragale Queue-de-Renard, Astragale Reglissier, Astragali, Astragalo, Astragalus membranaceus, Astragalus mongholicus, Astragli membranceus* also known as *huáng qí, běi qí* or *huáng hua̅ huáng qí* is a flowering plant in the family Fabaceae. It is one of the 50 fundamental herbs used in traditional Chinese medicine. *Astragalus* has been asserted to be a tonic that can improve the functioning of the lungs, adrenal glands and the gastrointestinal tract, increase metabolism and sweating, promote healing, and reduce fatigue based on traditional Chinese medicine theory.

The soybean or soya bean or *Glycine max* is a species of legume native to East Asia, widely grown for its edible bean which has numerous uses. The plant is classed as an oilseed rather than a pulse by the UN Food and Agriculture Organization (FAO). The genus *Glycine* Willd. is divided into two subgenera, *Glycine* and *Soja*. The subgenus *Soja* (Moench) F.J. Herm. includes the cultivated soybean, *Glycine max* (L.) Merr., and the wild soybean, *Glycine soja* Sieb. & Zucc. Both species are annuals. *Glycine soja* is the wild ancestor of *Glycine max.* The subgenus *Glycine* consists of at least 25 wild perennial species: for example, *Glycine canescens* F.J. Herm. and *G*. *tomentella* Hayata. Perennial soybean (Neonotonia wightii) originated in Africa and is now a widespread pasture crop in the Tropics.

Soy foods have played an important role in the diets of many Asian countries for centuries. Postulated benefits include protection against coronary heart disease (CHD), certain forms of cancer, especially breast and prostate cancer, and osteoporosis, and the alleviation of hot flashes. Isoflavones are polyphenolic compounds found predominantly in legumes (soy, chickpeas, lentils, and beans) and in red clover. According to the reports submitted by the National Cancer Institute of American, people who eat soybean products can reduce the incidence rate for prostate cancer.

However, none of *Astragalus membranaceus* and *Glycine max* are known or used for treating BHP. It is surprisingly found in accordance with the practice of this invention that an extract or powder of a herbal mixture comprising *Astragalus membranaceus* and *Glycine max* is effectively used as an add-on therapy for BHP treatment, as further defined in the claims.

In some embodiments, provided herein are compositions suitable for use in treating benign prostatic hyperplasia (BPH) in a subject comprising an extract or powder of a herbal mixture comprising *Astragalus membranaceus* and *Glycine max,* and an anti-BPH agent, as defined in the claims. The extract or powder of herbal mixture comprising *Astragalus membranaceus* and *Glycine max* used as an add-on therapy provides therapeutic benefit to a subject being treated for BPH (*see* Examples 1-2).

In some embodiments, said extract or powder of *Astragalus membranaceus* comprises at least one, two, three, or four components selected from the group consisting of polysaccharides, triterpenoid saponins, flavonoids, and amino acids. In some embodiments, said polysaccharides comprise alfalfa polysaccharide-1 (APS-1), alfalfa polysaccharide-1(APS-2), or the like, or combinations thereof. In some embodiments, said triterpenoid saponins comprise acetylastragaloside I, astragaloside I, astragaloside II, astragaloside IV, astramembrannin II, or the like, or combinations thereof. In some embodiments, said extract or powder of *Glycine max* comprises Genistein, Daidzein, or the like, or combination thereof. In certain embodiments, said extract or powder of *Astragalus membranaceus* and *Glycine max* is in a ratio of 1:2 to 24:1 by weight. In a particular embodiments, said extract or powder of *Astragalus membranaceus* and *Glycine max*is in a ratio of 24:1 by weight.

In some embodiments, the compositions described herein for use in treating benign prostatic hyperplasia (BPH) in a subject reduce prostate volume. In certain embodiments, said composition reduces the severity of BPH based on International Prostate Symptom Score (IPSS) or Quality of Life (QoL) index. In certain embodiments, said composition improves urine flow rate or nocturia of the subject.

In some embodiments, provided herein are compositions for use in treating benign prostatic hyperplasia (BPH) in a subject comprising an extract or powder of a herbal mixture, with an anti-BPH agent to said subject wherein said extract or powder of a herbal mixture comprises *Astragalus membranaceus* and *Glycine max,* as defined in the claims. The extract or powder of herbal mixture comprising *Astragalus membranaceus* and *Glycine max* used as an add-on therapy provides therapeutic benefit to a subject being treated for BPH (*see* Examples 1-2). In some embodiments, said extract or powder of *Astragalus membranaceus* comprises at least one, two, three, or four components selected from the group consisting of polysaccharides, triterpenoid saponins, flavonoids, and amino acids. In some embodiments, said polysaccharides comprise alfalfa polysaccharide-1 (APS-1), alfalfa polysaccharide-1(APS-2), or the like, or combinations thereof. In some embodiments, said triterpenoid saponins comprise acetylastragaloside I, astragaloside I, astragaloside II, astragaloside IV, astramembrannin II, or the like, or combinations thereof. In some embodiments, said extract or powder of *Glycine max* comprises Genistein, Daidzein, or the like, or combination thereof. In certain embodiments, said extract or powder of *Astragalus membranaceus* and *Glycine max* is in a ratio of 1:2 to 24:1 by weight. In a particular embodiments, said *Astragalus membranaceus* and *Glycine max* is in a ratio of 24:1 by weight. In some embodiments, the method reduces prostate volume. In certain embodiments, said method reduces the severity of BPH based on International Prostate Symptom Score (IPSS) or Quality of Life (QoL) index. In certain embodiments, said method improves urine flow rate or nocturia of the subject.

In some embodiments provided herein is a composition for use in treating benign prostatic hyperplasia (BPH) in a subject comprising an extract or powder of a herbal mixture as an add-on therapy in a subject wherein said herbal mixture comprises *Astragalus membranaceus* and *Glycine max.* (*see* Examples 1-2), as defined in the claims. In certain embodiments, said extract or powder of *Astragalus membranaceus* comprises at least one, two, three, or four components selected from the group consisting of polysaccharides, triterpenoid saponins, flavonoids, and amino acids. In certain embodiments, said polysaccharides comprise alfalfa polysaccharide-1 (APS-1), alfalfa polysaccharide-1(APS-2), or combinations thereof. In certain embodiments, said triterpenoid saponins comprise acetylastragaloside I, astragaloside I, astragaloside II, astragaloside IV, astramembrannin II, or combinations thereof. In certain embodiments, said extract or powder of *Glycine max* comprises Genistein, Daidzein or combination thereof. In certain embodiments, said extract or powder of *Astragalus membranaceus* and *Glycine max* is in a ratio of 1:2 to 24:1 by weight. In certain embodiments, said extract or powder of herbal mixture is administered orally.

The anti-BPH agent is tamsulosin or doxazosin, or its pharmaceutical salt, or solvate thereof. In some embodiments provide a composition comprising an extract or powder of a herbal mixture of *Astragalus membranaceus* and *Glycine max* for use in treating benign prostatic hyperplasia (BPH) as an add-on therapy, as defined in the claims. In some embodiments, said extract or powder of herbal mixture is suitable for administration orally. In some embodiments, the composition comprising an extract or powder of a herbal mixture of *Astragalus membranaceus* and *Glycine max* is used as an add-on therapy with an anti-BPH agent described herein. (*see* Examples 1-2)
An "anti-BPH" agent refers to an agent or substance that has a beneficial effect on the cause or symptoms of BPH either alone or in combination with a herbal mixture disclosed herein. In the present invention, the anti-BPH agent is an alpha-blocker.

As used herein, the term "alpha blockers" refers to any agents that block chemical activity (antagonist) at the alpha receptors, sites that respond to adrenaline-like substances, e.g., doxazosin (Cardura®, Pfizer, Inc.) or terazosin (Hytrin®, Abbott Laboratories). An alpha blocker may also be referred to as alpha adrenergic antagonist, alpha adrenergic blocking agent or alpha adrenergic blocker.

Examples of alpha blockers include, but are not limited to, doxazosin, terazosin, alfuzosin, phentolamine, phenoxybenzamine, prazosin, tamsulosin, and silodosin, or its pharmaceutically acceptable salt, or solvate thereof.

In the present invention, the alpha blocker is tamsulosin or doxazosin, or its pharmaceutically acceptable salt, or solvate thereof.

Other anti-BPH agents disclosed herein are 5-alpha reductase inhibitors. As used herein, "5-alpha reductase inhibitors" are a class of drugs with antiandrogen effects. These agents inhibit the enzyme 5α-reductase, which is involved in the metabolic transformations of a variety of endogenous steroids. 5α-reductase inhibition is most known for preventing conversion of testosterone, the major androgen sex hormone, to the more potent dihydrotestosterone (DHT), in androgen-associated disorders.

Examples of 5-alpha reductase inhibitors include, but are not limited to, dutasteride, epristeride, finasteride, izonsteride, turosteride, Ganoderic acid A, AS-601811, FK143, TF-505, and pharmaceutically acceptable salts, or solvates thereof.

In the present invention, the anti-BPH agent is tamsulosin or doxazosin, or its pharmaceutical acceptable salt or solvate.

### Certain Pharmaceutical and Medical Terminology

Unless otherwise stated, the following terms used in this application, including the specification and claims, have the definitions given below. It must be noted that, as used in the specification and the appended claims, the singular forms "a," "an" and "the" include plural referents unless the context clearly dictates otherwise. Unless otherwise indicated, conventional methods of mass spectroscopy, NMR, HPLC, protein chemistry, biochemistry, recombinant DNA techniques and pharmacology are employed. In this application, the use of "or" or "and" means "and/or" unless stated otherwise. Furthermore, use of the term "including" as well as other forms, such as "include", "includes," and "included," is not limiting. The section headings used herein are for organizational purposes only and are not to be construed as limiting the subject matter described.

The terms "co-administration" or the like, as used herein, are meant to encompass administration of the selected therapeutic agents to a single patient, and are intended to include treatment regimens in which the agents are administered by the same or different route of administration or at the same or different time.

The term "diluent" refers to chemical compounds that are used to dilute the compound of interest prior to delivery. Diluents can also be used to stabilize compounds because they can provide a more stable environment. Salts dissolved in buffered solutions (which also can provide pH control or maintenance) are utilized as diluents in the art, including, but not limited to a phosphate buffered saline solution.

The terms "effective amount" or "therapeutically effective amount," as used herein, refer to a sufficient amount of an agent or a compound being administered which will relieve to some extent one or more of the symptoms of the disease or condition being treated. The result can be reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in disease symptoms. An appropriate "effective" amount in any individual case may be determined using techniques, such as a dose escalation study.

The terms "enhance" or "enhancing," as used herein, means to increase or prolong either in potency or duration a desired effect. Thus, in regard to enhancing the effect of therapeutic agents, the term "enhancing" refers to the ability to increase or prolong, either in potency or duration, the effect of other therapeutic agents on a system. An "enhancing-effective amount," as used herein, refers to an amount adequate to enhance the effect of another therapeutic agent in a desired system.

The term "combination" or "add-on" as used herein, means a product that results from the mixing or combining of more than one active ingredient and includes both fixed and non-fixed combinations of the active ingredients. The term "fixed combination" means that the active ingredients, e.g. a herbal mixture (e.g., invention Composition **1**, or the like described herein) and a co-agent, are both administered to a patient simultaneously in the form of a single entity or dosage. The term "non-fixed combination" means that the active ingredients, e.g. a herbal mixture (e.g., invention Composition **1**, or the like described herein) and a co-agent, are administered to a patient as separate entities either simultaneously, concurrently or sequentially with no specific intervening time limits, wherein such administration provides effective levels of the two compounds in the body of the patient. The latter also applies to cocktail therapy, e.g. the administration of three or more active ingredients.

In some embodiments, said extract or powder of herbal mixture, and said anti-BPH agent is administered separately, simultaneously or sequentially.

The term "subject" or "patient" encompasses mammals. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. In one embodiment, the mammal is a human.

The terms "treat," "treating" or "treatment," as used herein, include alleviating, abating or ameliorating at least one symptom of a disease or condition, preventing additional symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically.

### Routes of Administration and Dosage

Suitable routes of administration include, but are not limited to, oral, intravenous, rectal, aerosol, parenteral, ophthalmic, pulmonary, transmucosal, transdermal, vaginal, otic, nasal, and topical administration. In addition, by way of example only, parenteral delivery includes intramuscular, subcutaneous, intravenous, intramedullary injections, as well as intrathecal, direct intraventricular, intraperitoneal, intralymphatic, and intranasal injections.

In certain embodiments, an anti-BPH agent is for administration in a local rather than systemic manner, for example, via injection of the compound directly into an organ, often in a depot preparation or sustained release formulation. In specific embodiments, long acting formulations are for administration by implantation (for example subcutaneously or intramuscularly) or by intramuscular injection. Furthermore, in other embodiments, the drug is delivered in a targeted drug delivery system, for example, in a liposome coated with organ-specific antibody. In such embodiments, the liposomes are targeted to and taken up selectively by the organ. In yet other embodiments, the compound as described herein is provided in the form of a rapid release formulation, in the form of an extended release formulation, or in the form of an intermediate release formulation. In yet other embodiments, the compound described herein is for topical administration.

In some embodiments, an anti-BPH agent is for parenteral or intravenous administration. In other embodiments, an anti-BPH agent is for administration by injection. In some embodiments, an anti-BPH agent is for oral administration.

In the case wherein the patient's condition does not improve, upon the doctor's discretion the administration of the compounds may be chronical, that is, for an extended period of time, including throughout the duration of the patient's life in order to ameliorate or otherwise control or limit the symptoms of the patient's disease or condition. In the case wherein the patient's status does improve, upon the doctor's discretion the administration of the compounds may be given continuously or temporarily suspended for a certain length of time (*i.e*., a "drug holiday").

The foregoing ranges are merely suggestive, as the number of variables in regard to an individual treatment regime is large, and considerable excursions from these recommended values are not uncommon. Such dosages may be altered depending on a number of variables, not limited to the activity of the compound used, the disease or condition to be treated, the mode of administration, the requirements of the individual subject, the severity of the disease or condition being treated, and the judgment of the practitioner.

In some embodiments, the extract or powder of herbal mixture described herein, and the anti-BPH agent is administered separately, simultaneously or sequentially.

In some embodiments, each of the extract or powder of herbal mixture, and the anti-BPH agent are independently administered orally, parenterally intravenously or by injection. In certain embodiments, the extract or powder of herbal mixture is administered orally. Toxicity and therapeutic efficacy of such therapeutic regimens can be determined by standard pharmaceutical procedures in cell cultures or experimental animals, including, but not limited to, for determining the LD₅₀ (the dose lethal to 50% of the population) and the ED₅₀ (the dose therapeutically effective in 50% of the population). The dose ratio between the toxic and therapeutic effects is the therapeutic index and it can be expressed as the ratio between LD₅₀ and ED₅₀. Compounds exhibiting high therapeutic indices are preferred. The data obtained from cell culture assays and animal studies can be used in formulating a range of dosage for use in human. The dosage of such compounds lies preferably within a range of circulating concentrations that include the ED₅₀ with minimal toxicity. The dosage may vary within this range depending upon the dosage form employed and the route of administration utilized.

### General Consideration for Add-on Therapy (A Type of Combination Treatments)

In general, the compositions described herein and, in embodiments where an add-on therapy or combinational therapy is employed based on the mode of action described herein, other agents do not have to be administered in the same pharmaceutical composition, and in some embodiments, because of different physical and chemical characteristics, are administered by different routes. In some embodiments, the initial administration is made according to established protocols, and then, based upon the observed effects, the dosage, modes of administration and times of administration is modified by the skilled clinician.

In some embodiments, therapeutically-effective dosages vary when the drugs are used in treatment combinations. Combination treatment further includes periodic treatments that start and stop at various times to assist with the clinical management of the patient. For combination therapies described herein, dosages of the co-administered compounds vary depending on the type of co-drug employed, on the specific drug employed, on the disease, disorder, or condition being treated and so forth.

It is understood that in some embodiments, the dosage regimen to treat, prevent, or ameliorate the condition(s) for which relief is sought, is modified in accordance with a variety of factors. These factors include the disorder from which the subject suffers, as well as the age, weight, sex, diet, and medical condition of the subject. Thus, in other embodiments, the dosage regimen actually employed varies widely and therefore deviates from the dosage regimens set forth herein.

It is understood that in some embodiments, the dosage regimen to treat, prevent, or ameliorate the condition(s) for which relief is sought, is modified in accordance with a variety of factors. These factors include the disorder from which the subject suffers, as well as the age, weight, sex, diet, and medical condition of the subject. Thus, in other embodiments, the dosage regimen actually employed varies widely and therefore deviates from the dosage regimens set forth herein.

### Examples

### Example 1. Herbal materials and preparation of herbal mixture extracts

*Astragalus membranaceus* extracts (e.g., from any suitable parts of the plant such as leaf, stem, root, etc.), which consists of four major ingredients, namely (1) aminoacid (major ingredient of protein), (2) polysaccharides (such as alfalfa polysaccharide-1 (APS-1), alfalfa polysaccharide-1(APS-2), or the like), (3) saponins (e.g., triterpenoid saponins such as acetylastragaloside I, astragaloside I, astragaloside II, astragaloside IV, astramembrannin II, or the like), and (4) flavonoids (increasing estrogen to suppress androgen). The study composition (i.e., Umooze), contains *Astragalus membranaceus* extracts and *Glycine max* in the combination of 1:2∼24:1 by weight. An exemplary Composition **1** containing *Astragalus membranaceus* extracts and *Glycine max* in the ratio of 960:40 in one 500 mg tablet was used in a clinical trial disclosed herein.

The decoction of Composition **1** (i.e. Umooze), used in this study was prepared as lyophilized powder, or an extraction (aqueous or organic solvents) from *Astragalus membranaceus* (Huang Qi) and *Glycine max.* Specifically, the following non limited ingredients are identified in the lyophilized powder, or the extraction of *Astragalus membranaceus*: (1) polysaccharides: APS-I and APS-II, (2) triterpenoid saponins: acetylastragaloside I, astragaloside I, astragaloside II, astragaloside IV, and/or astramembrannin II, (3) flavonoids (e.g., quercetin), and (4) amino acids such as asparagines. The following non limited ingredients are identified in the lyophilized powder, or the extraction of *Glycine max*: Genistein, and/or Daidzein.

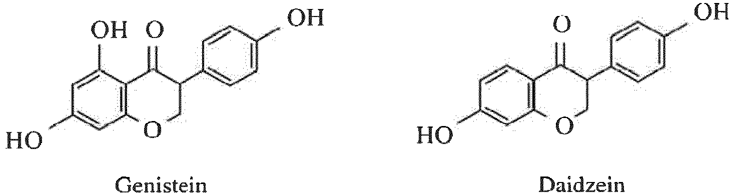

### Example 2: Clinical Study of Composition 1 as an add-on therapy on Benign Prostatic Hyperplasia Patients

This study was a randomized, two-regimen, placebo-controlled, parallel study. Subjects were randomized to one of the two groups to receive the add-on Composition **1** (i.e. Umooze) or placebo with its own medication. The add-on study product (i.e., Composition **1**) was administrated twice daily for 56 days. Primary objective is to evaluate the improvement in symptoms of Benign Prostatic Hyperplasia (BPH) according to the International Prostate Symptom Score (IPSS) and Quality-of-Life Index (QoL) index after add-on therapy of Composition **1** twice daily administration for 56 days in patients with BPH.

The International Prostate Symptom Score (I-PSS) is based on the answers to seven questions concerning urinary symptoms and one question concerning quality of life. Each question concerning urinary symptoms allows the patient to choose one out of six answers indicating increasing severity of the particular symptom. The answers are assigned points from 0 to 5. The total score can therefore range from 0 to 35 (asymptomatic to very symptomatic). The questions refer to the following urinary symptoms: Incomplete emptying, Frequency, Intermittency, Urgency, Weak Stream, Straining, and Nocturia.

It is known in the art that the feeling of incomplete bladder emptying, two urination intervals of less than 2 hours, weak urine stream and nocturnal urination frequency showed a statistically significant correlation with quality of life Index.

Secondary objective is to assess the improvement in uroflowmetry (UFR) measure of maximum urinary flow rate (Qmax), postvoided residual volume (PVR), prostate volume and serum prostate specific antigen (PSA) after add-on therapy of Umooze twice daily administration for 56 days in patients with BPH.

When evaluating patients with voiding dysfunction, noninvasive tests such as uroflowmetry (UFR) and measurement of postvoid residual urine volume (PVR) can help to determine whether additional testing is warranted. Uroflowmetry measures urine voided per unit time, which is usually expressed as milliliters per second. The International Continence Society has standardized certain objective measurements to be recorded during uroflow measurement. Measurement of postvoid residual urine volume (PVR), the amount of residual urine in the bladder after a voluntary void, is another noninvasive screening test for evaluating voiding dysfunction. PVR can be measured by 2 methods: catheterization or bedside bladder ultrasonography. Although both methods have advantages, the convenience, efficiency, and safety of bladder ultrasound makes its use beneficial in a wide variety of populations, including hospitalized patients, children, and the elderly. More recently, bladder ultrasound has been used for other procedures, such as suprapubic aspiration, evaluation of intravesical masses, and to determine bladder wall thickness and bladder wall mass, both of which have been associated with outflow obstruction.

A maximum urinary flow rate (Qmax) is used to assess urine flow rate. It should be used as a baseline before embarking on any treatment, whether medical or surgical. The maximal flow rate (Qmax) is the single best measurement but a low Qmax does not help to differentiate between obstruction and poor bladder contractility. A Qmax value over 15 mL/second is usually considered normal. A Qmax below 7 mL/second is accepted as low. Results can vary according to effort and volume and so the usual compromise is to obtain at least two readings with at least 150 mL of urine each time.

Prostate-specific antigen (PSA), also known as gamma-seminoprotein or kallikrein-3 (KLK3), is a glycoprotein enzyme encoded in humans by the *KLK3* gene. PSA is a member of the kallikrein-related peptidase family and is secreted by the epithelial cells of the prostate gland. PSA is produced for the ejaculate, where it liquefies semen in the seminal coagulum and allows sperm to swim freely. It is also believed to be instrumental in dissolving cervical mucus, allowing the entry of sperm into the uterus. PSA is present in small quantities in the serum of men with healthy prostates, but is often elevated in the presence of prostate cancer or other prostate disorders. PSA is not a unique indicator of prostate cancer, but may detect benign prostatic hyperplasia.

The study is expected to be completed when a total number of valuable subjects reached at least 36. Forty-one (41) subjects were enrolled into this study and only thirty-six (36) subjects completed the entire study. Each subject received either Composition **1** (Umooze) or placebo in the morning (e.g., at 07:30) and in the evening (e.g., at 19:30) on study Day for a continuous 56-day. Each oral dose of Composition **1** was given as two tablets with 240+20 mL of water. A subject will be required to make a total of 6 visits in the study period.

### Inclusion Criteria

Subjects must fulfill all of the following criteria to be eligible to enter the study:
1. Males aged ≥ 40 years old.
2. Screened by inquiry and diagnosed as BPH based on the result of a digital rectal examination (DRE) or transrectal ultrasonography (TRUS).
3. Prostate volume ≥ 20 cm³.
4. Has complained of voiding symptoms related to BPH.
5. Has an IPSS ≥ 13 or an UFR measure of Qmax ≤ 15 mL/sec together with a voided volume ≥ 150 mL.
6. Serum PSA < 4 ng/mL.
7. Has been treated with medication for BPH.
8. Informed consent form signed.

### Exclusion Criteria

Subjects with any of the following criteria will be excluded from the study:
1. Sensitivity to study product.
2. Had received prostatic surgery for BPH in the past.
3. Hard nodule found by DRE.
4. Ongoing neurogenic bladder, urinary tract infection, bladder stone, urethral stricture, bladder cancer, prostate cancer, severe liver dysfunction, severe renal dysfunction or severe cardiovascular disease.
5. Patient has clinically significant physical disability or abnormal findings on physical examination or laboratory testing judged by the investigator or co-investigator.
6. Participation of any clinical investigation during the last 30 days.
7. Individuals are judged by the investigators or co-investigator to be undesirable as subjects.

During the study period, the events and time schedule was as follows:

| **Period** | **Screening/Baseline** | **Treatment** | | | | |
|---|---|---|---|---|---|---|
| Visit | V 1 | V 2 | V 3 | V 4 | V 5 | V 6 |
| Day (D)* | D -7 | D 0 | D 14 | D 28 | D 42 | D 56 |
| Week (W) | W -1 | W 0 | W 2 | W 4 | W 6 | W 8 |
| Medical History | x | | | | | |
| Clinical Examination | x | | | | | x |
| Laboratory Measurement | x | | | | | x |
| IPSS Assessment | x | | | x | x | x |
| UFR Measurement | x | | | x | | x |
| PVR Measurement | x | | | | | x |
| Prostate Volume Measu- | x | | | | | x |
| Serum PSA Level | x | | | | | x |
| Study Product | | x | x | x | x | |
| Vital Sign Measurement | x | | | x | | x |
| Safety Measurement | | | x | x | x | x |

| | | | | | | |
|---|---|---|---|---|---|---|
| *: An acceptable range at each visit will be within ±7 day. | | | | | | |

### INVESTIGATION PLAN

Regimen A -CONTROL
   Name: placebo
Regimen B - TEST
   Name: Composition **1**
   Major element: *Astragalus* radix Extracts 480 mg + *Glycine max* Extracts 20 mg Dosage form: Tablet

### Investigation Regimen of Study Product

The study products were provided in sufficient amount before next visit on study day 0, 14, 28 and 42. The IPSS assessment was assessed at baseline, 28^{th}, 42^{nd} and 56^{th} day. The UFR measurement and vital sign measurement were performed at baseline, 28^{th} and 56^{th} day. The PVR, prostate volume, serum PSA and laboratory measurement were performed at baseline and 56^{th} day.

### Duration of Study

The study period for each subject will at least for 56 days. A subject will be required to make a total of 6 visits in the study period.

### Concomitant Treatments

**Concomitant Medications:** Patients' routinely used medications or treatments which are judged by the investigator or co-investigator as not to affect the benefit and safety assessments in this study are permitted. Any use of drugs will be documented in the case report forms (CRF), specifying the substance, dose, time and reason for use.

### STUDY MEASUREMENT AND VARIABLES

**Prestudy Screening:** Subjects give the signed informed consent form will be screened. The tasks planned for completion at every visit in study period, are shown below.

### Screening/Baseline Phase (Visit 1; Day -7; Week -1)

The following data will be obtained or procedures will be done during this visit:
1. Evaluate inclusion/exclusion criteria
2. Assess medical history
3. Clinical Examination
4. Record demographics
5. Laboratory measurement
6. IPSS assessment
7. UFR measurement
8. PVR measurement
9. Prostate volume measurement
10. Serum PSA level
11. Complete informed consent

### Treatment Phase

**Visit 2 (Day 0; Week 0):** At visit 2, those enrolled subjects will receive the following series of tasks:
   1. Receive the provided Composition **1** or placebo for 14-day dose
   2. Oral administration of Composition **1** or placebo twice daily for 14 days
   3. Record the concomitant used medications
**Visit 3 (Day 14; Week 2):** The tasks that should be carried out at visit 3 are the following:
   1. Receive the provided Composition **1** or placebo for 14-day dose
   2. Oral administration of Composition **1** or placebo twice daily for 14 days
   3. Record the concomitant used medications
   4. Safety measurement
**Visit 4 (Day 28; Week 4):** The tasks that should be carried out at visit 4 are the following:
   1. IPSS assessment
   2. UFR measurement
   3. Receive the provided Umooze or placebo for 14-day dose
   4. Oral administration of Umooze or placebo twice daily for 14 days
   5. Assess vital signs (heart rate, blood pressure and body temperature)
   6. Record the concomitant used medications
   7. Safety measurement
**Visit 5 (Day 42; Week 6):** The tasks that should be carried out at visit 5 are the following:
   1. IPSS assessment
   2. Receive the provided Umooze or placebo for 14-day dose
   3. Oral administration of Umooze or placebo twice daily for 14 days
   4. Record the concomitant used medications
   5. Safety measurement
**Visit 6 (Day 56; Week 8):** The tasks that should be carried out at visit 6 are the following:
   1. IPSS assessment
   2. UFR measurement
   3. PVR measurement
   4. Prostate volume measurement
   5. Serum PSA level
   6. Assess vital signs (heart rate, blood pressure and body temperature)
   7. Safety measurement
   8. Clinical Examination
   9. Laboratory measurement

### Clinical and Laboratory Measurement

**Clinical Examination:** The clinical examination includes vital signs and physical examination. Blood pressure and heart rate in the sitting position, body weight and height will be measured. The diagnosis of BPH would be based on the result of a DRE or TRUS.

**Laboratory Measurement:** A blood sample will be taken at prestudy screening and visit 5 in order to perform the laboratory measurements. If subjects have performed any item of the laboratory tests within two month before entering the study, the item will not be performed at prestudy screening. The abnormal observation will be recorded on the CRFs and the evaluation of clinical significance will be judged by the investigators. For early termination, the most possible efforts will be made to encourage the subject to complete the final laboratory test.

Laboratory measurements are as follows:
- Hematology:: Hemoglobin, hematocrit, WBC count with differential, RBC count and platelet count.
- Biochemistry:: SGOT (AST), SGPT (ALT), creatinine, BUN and total bilirubin.

**Benefit Measurement:** The improvement in symptoms of BPH assessed according to the IPSS and QoL index will be evaluated. The International Prostate Symptom Score (IPSS) is a validated 7-item urinary symptom severity scale. This symptom score is a 7-item questionnaire designed for patient self-administration. The answers are assigned points from 0 to 5, indicating increasing severity. The total score can therefore range from 0 to 35 points (asymptomatic to very symptomatic). Patients can be classified as follows: ≤ 7 mildly symptomatic; 8∼19 moderately symptomatic; 20∼35 severely symptomatic. The Quality-of-Life (QoL) index is a single question with scores of 0∼6 point and corresponding to the assessment index ranges from delighted to terrible. The improvement in uroflowmetry (UFR) measure of maximum flow rate (Qmax), postvoided residual volume (PVR), prostate volume and serum PSA level will also be observed. The UFR measure of Qmax is to determine the degree of urinary difficulty.

Benefit will be evaluated at baseline and subsequently on study day 28, 42 and 56. The primary objective is to evaluate the mean change in IPSS and QoL index at the endpoint visit from baseline after add-on therapy of Composition **1.** The secondary objective is to measure the mean change in Qmax, PVR, prostate volume and serum PSA at the endpoint visit from baseline after add-on therapy of Composition **1.** The difference of the mean change in symptoms of BPH (assessed by IPSS and QoL index) and improvement in Qmax, PVR, prostate volume and serum PSA during study period after add-on therapy of Composition **1** or these differences between placebo therapy vs. add-on therapy will also be assessed.

**Safety Measurement:** Safety will be evaluated by the clinically significant changes in terms of:
1. Physical examination
2. Vital signs
3. Laboratory examination parameters
4. Incidence of adverse events

An Adverse Event/Experience (AE) is any untoward medical occurrence in a patient or clinical investigation subject administered a pharmaceutical product which does not necessarily have a causal relationship with this treatment. An AE can therefore be any unfavorable and unintended sign, symptom or disease temporally associated with the use of the study product, whether or not considered related to the study product.

**Adverse Events (AEs):** Adverse events (AE) will be described in the CRF by type, time of onset, duration and course and their intensity and relationship to the study product will be judged. Special reporting procedures apply to AEs which are considered as serious. They must be immediately reported to a representative of the sponsors. Some definitions are given in the following:
A Serious Adverse Event (SAE) is any untoward medical occurrence that at any dose:
▪ Results in death
▪ Is life-threatening
▪ Results in persisting or significant disability/incapacity
▪ Requires hospitalization or prolongs hospitalization
▪ Is a congenital anomaly/birth defect
▪ May jeopardize the patient or may require intervention to prevent on or the other outcomes listed above

A Suspected Unexpected Serious Adverse Reaction (SUSAR) is defined as an adverse reaction, the nature or severity of which is not consistent with the applicable product information. As soon as new information about the SAE or SUSAR becomes known the investigator has to forward it without any delay to the project manager of the sponsor and the Institutional review board/Ethics Committee (IRB/EC). This applies to the follow-up and the final report and all medical records associated with it. A copy of the updated CRF will be forwarded to the project manager of the sponsor and the IRB/EC as soon as possible.

**Vital Signs:** Heart rate, systolic and diastolic blood pressure and body temperature will be measured and recorded in sitting position at the following times: Study Day -7, 28 and 56. **Post Study Examinations:** Within 7 days after completion of the clinical part, a final examination including hematology and biochemistry tests as carried out for subject inclusion will be performed in order to determine possible changes in the subject's state of health.

### DATA QUALITY ASSURANCE

**Quality assurance system:** Standard operating procedures are available for all activities relevant to the quality of the study. Results of these audits as well as any objections will be reported directly to the management.

**Monitoring:** The sponsor or a representative of the sponsor may at any time demand information on the progress of the study by phone or in writing, or may visit the study site in order to monitor study procedures and to review the original study documents and subject's data. **Documentation:** Standardized CRF will be used to document all data collected during the course of the study. All entries will be completed in black ball-point pen. Corrections will be made in such a way that the original entry is not obscured. All corrections will be dated and initialed.

For source data verification, at least the following information will be recorded as source data in separate documents/file and transferred to the CRF:
▪ Date of subject's written informed consent
▪ The fact that the subject is in a clinical study and the study number (screening and subject no.)
▪ Questionnaire (medical and personal history)
▪ Body weight, height, vital signs result at screening
▪ Results of screening and follow-up test (laboratory examination)
▪ Study product using

All other data will be directly recorded on the CRF and regarded as source data, i.e. no prior written or electronic data is available. Laboratory data will be transferred with a printout form provided by the contract laboratory.

### DATA EVALUATION AND STATISTICAL CONSIDERATION

**Subject Numbers:** A minimum of 18 evaluable (intention-to-treat) patients with BPH will be required.

### Statistical Analysis:

**Continuous Variable:** The raw data will be summarized as the number of observations, mean, median, standard deviation, minimum, and maximum on each-visit basis. Continuous variables will be analyzed by t-test or Wilcoxon Rank Sum test.

**Categorical Variable:** The raw data will be summarized as the number of observations, frequency of each class on each-visit basis. Chi-square test, Fisher's exact test or CMH test will be adopted if needed.

### Analysis Population

Data analyses and summaries of the safety endpoints will be performed for the following populations:
Intention-to-Treat (ITT) Population --- all subjects who receive at least once of study product and have at least one post-baseline assessment for the objective variable regardless of their compliance with the protocol.

Per-protocol (PP) Population --- All subjects who using the study product and who complete the study without any major protocol deviation.

Safety Population --- all subjects who receive at least once of the study product. Safety endpoints will be analyzed on the safety population.

Analyses will conduct using a Last Observation Carried Forward (LOCF) for the missing value. Primary objective will be analyzed based primarily on the ITT population, and the LOCF approach will be used in missing value. The safety evaluation will be based on the ITT and PP populations but the final analysis will be derived from the ITT population. **Assessment of Safety:** Safety will be assessed using the following:
1. Physical examination
2. Vital signs
3. Laboratory assessment
4. Adverse event monitoring

Safety evaluation will include changes in laboratory data, vital signs, physical examination parameters and all adverse events that may have happened during the study.

### Clinical Trial Results

Forty-one (41) subjects were enrolled into the study. During the study periods, several subjects withdrew from the study due to personal reason, a few subjects withdrew from the study due to administrative reason, and they were replaced because the expected number of subjects to complete the whole study (at least 36) should be enough. Therefore, the total numbers of subjects completed the entire study is 36. The subjects were randomized in a 1:1 ratio to receive the add-on Umooze (i.e., Composition **1**) or placebo with their own medication. Only the data obtained from the subjects who completed the whole study are reported.

The primary objective is to evaluate the mean change in IPSS and QoL index at the endpoint visit from baseline after add-on therapy of Umooze.

For the Umooze group, the mean of difference is -3.39 (SD: 6.11), and for the placebo group, the mean of difference is -5.94 (SD: 8.11). When testing their difference by Wilcoxon rank sum test and Student-t test, the P-values of these two statistic test are 0.4752 and 0.2935, respectively. There was no statistically significant difference between Umooze group and placebo group.

The study product (Umooze or placebo) is given as add-on therapy in BPH, therefore the evaluation of benefit may be affected by the original medication. In CRF's records, each subject received distinct drugs or dose which depend on their investigator's prescription. For all subject's medical history in this study, there are one subject receiving Harnalidge® (Tamsulosin hydrochloride) 0.1 mg once daily (QD), five subjects receiving Harnalidge® 0.2 mg QD, twenty-two subjects receiving Harnalidge® 0.4 mg QD and eight subjects receiving Doxaben® (Doxazosin mesilate) 4 mg or Doxaben® XL 4 mg QD for BPH treatment.

In order to dismiss the impact of distinct drugs on the benefit of the add-on therapy, the mean change in IPSS at the endpoint visit from baseline is assessed again only in twenty-two (22) subjects who received Harnalidge® 0.4 mg QD treatment for BPH. The mean change in IPSS at each visit from baseline in these twenty-two (22) subjects (11 in Umooze group and 11 in placebo group) are analyzed. Similarly, in order to dismiss the impact of distinct drugs on the benefit of the add-on therapy, the mean change in QoL index at each visit from baseline is assessed again only in 22 subjects who were received Harnalidge® 0.4 mg QD treatment for BPH. The mean change in QoL index at each visit from baseline in these twenty-two (22) subjects (11 in Umooze group and 11 in placebo group) are analyzed.

The secondary objective is to measure the mean change in Qmax, PVR, prostate volume and serum PSA at the endpoint visit from baseline after add-on therapy of Umooze.

The IPSS of all subjects at each visit were transferred into the degree of symptom according to the classification of following: ≤7 mildly symptomatic; 8∼19 moderately symptomatic; 20∼35 severely symptomatic. The number of subjects of the three degree of symptom at each visit in Umooze group and placebo group could be expressed as percentage (%) and assessed. The percentage (%) of mildly and moderately symptomatic at visit 1 (baseline) in Umooze group and placebo group are 38.9% and 25.0%, respectively. After add-on therapy for 28 day, the percentage (%) of mildly and moderately symptomatic at visit 4 (28^{th} day) in Umooze group and placebo group are 50.0% and 44.4%, respectively. After add-on therapy for 42 day, the percentage (%) of mildly and moderately symptomatic at visit 5 (42^{th} day) in Umooze group and placebo group are 50.0% and 38.9%, respectively. After add-on therapy for 56 day, the percentage (%) of mildly and moderately symptomatic at visit 6 (56^{th} day) in Umooze group and placebo group are 47.2% and 41.7%, respectively.

The quantiles of difference in IPSS, QoL index, Qmax, PVR, prostate volume and serum PSA after add-on therapy of Umooze twice daily administration in patients with BPH also plotted in Box plot form. The Box-plot is a convenient way of graphically depicting groups of numerical data through their quantiles (25%, 50% and 75%). The 25%, 50% and 75% quantiles of difference in IPSS, show in FIGs. 1∼6, were slightly higher in Umooze group compared to the placebo group except for FIG. 4. The median (50% quantile) of difference in QoL index, show in FIGs. 7∼12, were slightly lower in Umooze group compared to the placebo group except for FIGs. 9 and 12. The median (50% quantile) of difference in Qmax, show in FIGs. 13∼14, was slightly lower in Umooze group compared to the placebo group except for FIG. 14. The 25%, 50% and 75% quantiles of difference in PVR, show in FIG. 15, was lower in Umooze group compared to the placebo group. The Box plot of prostate volume and PSA are show in FIGs. 16 and 17, respectively.

On the primary objective of this study, result of the mean change in IPSS and QoL index at the endpoint visit from baseline, was described as above. The mean difference of QoL index at endpoint visit from baseline in Umooze group and placebo group were -0.61 ± 1.20 and - 0.56 ± 1.20, respectively. Therefore, it is evidenced that the mean difference of QoL index after Umooze add-on therapy at endpoint visit (56^{th} day) from baseline had a better decrease tendency compared with placebo add-on therapy. Besides, the mean difference of QoL index at visit 5 from baseline in Umooze group and placebo group were -0.50 ± 1.29 and 0.44 ± 1.29, respectively. It is evidenced that the mean difference of QoL index after Umooze add-on therapy at visit 5 (42^{th} day) from baseline had a decrease tendency compared with placebo add-on therapy.

The mean difference of IPSS at visit 5 from baseline in subjects with same medication for BPH treatment of Umooze group and placebo group were -4.18 ± 5.93 and -2.82 ± 6.81, respectively. Therefore, it can be concluded that the mean difference of IPSS after Umooze add-on therapy at visit 5 (42^{th} day) from baseline had a better decrease tendency compared with placebo add-on therapy.

The mean difference of QoL index at endpoint visit from baseline in Umooze group and placebo group were -0.82 ± 0.87 and -0.09 ± 0.70, respectively. The mean difference of QoL index at visit 5 from baseline in Umooze group and placebo group were -0.91 ± 1.04 and - 0.09 ± 0.94, respectively. According to the result of statistical analysis by Wilcoxon rank sum test, the mean change in QoL index after Umooze add-on therapy at visit 6 (56^{th} day) and visit 5 (42^{th} day) in subjects with same medication for BPH treatment was significantly decrease (P-value was 0.0350 and 0.0384, respectively) from baseline (visit 1) compared with placebo add-on therapy. It indicates that a better improvement in QoL index after Umooze add-on therapy for 42∼56 day in subjects with same medication for BPH treatment compared with placebo add-on therapy.

On the secondary objective of this study, result of the mean change in UFR measure of Qmax, PVR, prostate volume and serum PSA level at the endpoint visit from baseline, were described as above. The mean difference of Qmax at endpoint visit from baseline in Umooze group and placebo group were 2.88 ± 3.77 and 2.49 ± 5.46, respectively. Therefore, it can be concluded that the mean difference of Qmax after Umooze add-on therapy at endpoint visit (56^{th} day) from baseline had a higher increase tendency compared with placebo add-on therapy.

The mean difference of prostate volume at endpoint visit from baseline in Umooze group and the placebo group were -5.07 ± 6.1 and -1.17 ± 7.14, respectively. Therefore, it can be concluded that mean difference of prostate volume after Umooze add-on therapy at endpoint visit (56^{th} day) from baseline had a larger decrease tendency compared with placebo add-on therapy.

The mean difference of PVR at endpoint visit from baseline in Umooze group and placebo group were -8.39 ± 31.9 and 14.8 ± 32.8, respectively. Therefore, it can be concluded that the mean difference of PVR after Umooze add-on therapy at endpoint visit (56^{th} day) from baseline had a larger reduce tendency compared with placebo add-on therapy. In accordance with the result of statistical analysis by Wilcoxon rank sum test, the mean change in PVR after Umooze add-on therapy at visit 6 (56^{th} day) was significantly reduce (P-value was 0.0314) from baseline (visit 1) compared with placebo add-on therapy. It indicates that a better improvement in PVR after Umooze add-on therapy for 56 day in BPH patients compared with placebo add-on therapy.

In summary, the benefit of Umooze add-on therapy in the management of BPH is clearly shown. The mean change in QoL index at 42^{th} day and 56^{th} day were significant decrease after Umooze add-on therapy in patients with Harnalidge® 0.4 mg QD treatment for BPH. Therefore, the quality of life on BPH patients could be improve after Umooze add-on therapy for 42∼56 day. The mean change in PVR at 56^{th} day was significant reduce after Umooze add-on therapy in patients with BPH. The decrease in postvoided residual volume suggests a positive effect on obstruction. Therefore, Umooze add-on therapy could helps in effective evacuation of the bladder in patients with BPH.

The safety of Umooze add-on therapy was monitored throughout the study. During the clinical evaluation, heart rate, systolic and diastolic blood pressure and body temperature were measured and recorded in sitting position. The measured vital sign values of all subjects were within the normal range. No seriously adverse reaction was reported during the study.

In short as summarized in Table 1, it is clearly shown that the efficacies of UMOOZE on patients with BHP were:
1. Improved the mean change from baseline in IPSS at Day 28 (visit 4, all patients).
2. Improved the mean change from baseline in IPSS at Day 28 (visit 4, patients who fit Qmax ≦ 15mL/sec criteria).
3. Improved the mean change from baseline in IPSS at Day 28 (visit 4, patients take BPH drug less than 6 months, p ≦ 0.05).
4. Improved the mean change from baseline in IPSS at Day 28 (visit 4, patients take Harnalidge 0.4mg once daily for up to 6 months, p ≦ 0.05).
5. Improved the mean change from baseline in IPSS at Day 28 (visit 4, patients with non-Harnalidge 0.4mg treatment).
6. Improved the nocturia in patients with Qmax ≦ 15mL/sec at any stages of study.
7. Improved the mean change from baseline in QoL at Day 56 (visit 6, patients who fit IPSS criteria).
8. Improved the mean change from baseline in QoL at Day 56 (visit 6, patients take Harnalidge 0.4mg once daily, p ≦ 0.05).
9. Improved the mean change from baseline in QoL at Day 56 (visit 6, patients take Harnalidge 0.4mg once daily and fit IPSS criteria, p ≦ 0.05).
10. Improved the mean change from baseline in QoL at Day 56 (visit 6, patients take Harnalidge 0.4mg once daily and fit Qmax criteria).
11. Improved the mean change from baseline in QoL at Day 42 (visit 5, patients take Harnalidge 0.4mg once daily).
12. Improved the mean change from baseline in QoL at Day 42 (visit 6, patients take Harnalidge 0.4mg once daily and fit both Qmax ≦ 15mL/sec and IPSS criteria).
13. Improved the mean change from baseline in PVR at Day 56 (visit 6, all patients, p ≦ 0.05).
14. Improved the mean change from baseline in PVR at Day 56 (visit 6, patients who fit IPSS criteria).
15. Improved the mean change from baseline in PVR at Day 56 (visit 6, patients who fit Qmax ≦ 15mL/sec criteria, p ≦ 0.001).
16. Reduced the prostate volume at Day 56 (visit 6, all patients).
17. Reduced the prostate volume at Day 56 (visit 6, patients who fit IPSS criteria).
18. Reduced the prostate volume at Day 56 (visit 6, patients who fit Qmax ≦ 15mL/sec criteria, p ≦ 0.05).

**Table 1 summarizes the clinical trial results.**

| **Research on Benefit of Umooze as Add-on Therapy in Benign Prostatic Hyperplasia** | | |
|---|---|---|
| **Effectiveness** | **Moderate** | **Significant** |
| Difference of IPSS between V1 and V4(D28) | P=0.068 | |
| Difference of IPSS between V1 and V4 in fit Qmax | p=0.093 | |
| Difference of IPSS between V1 and V4 in BPH treatment for < 6ms | | p=0.012 |
| Difference of IPSS between V1 and V4 in Harnalidge 0.4mg treatment for < 6ms | | p=0.0459 |
| Difference of IPSS between V1 and V5 (D42) in non-Harnalidge 0.4mg treatment | p=0.091 | |
| Difference of IPSS between V1 and V4 in non-Harnalidge 0.4mg treatment | p=0.070 | |
| Difference of nocturia in IPSS from every visits to V1 in fit Qmax | p=0.086 | |
| Difference of QoL between V1 and V6 (D56) in fit IPSS | p=0.084 | |
| Difference of QoL between V1 and V6 in Harnalidge 0.4mg once daily treatment | | p=0.044 |
| Difference of QoL between V1 and V6 in Harnalidge 0.4mg treatment in fit IPSS | | p=0.028 |
| Difference of QoL between V1 and V6 in Harnalidge 0.4mg treatment in fit Qmax | p=0.056 | |
| Difference of QoL between V1 and V5 in Harnalidge 0.4mg once daily | p=0.068 | |
| Difference of QoL between V1 and V5 in Harnalidge 0.4mg in fit IPSS | p=0.056 | |
| Difference of QoL between V1 and V5 in Harnalidge 0.4mg in fit Qmax | p=0.085 | |
| Difference of PVR between V1 and V6 | | p=0.0314 |
| Difference of PVR between V1 and V6 in fit IPSS | p=0.062 | |
| Difference of PVR between V1 and V6 in fit Qmax | | p=0.00000129 |
| Difference of prostate volume between V1 and V6 | p=0.0518 | |
| Difference of prostate volume between V1 and V6 in fit IPSS | p=0.079 | |
| Difference of prostate volume between V1 and V6 in fit Qmax | | p=0.047 |

| | | |
|---|---|---|
| IPSS: International Prostate Symptom Score QoL: Quality-of-Life Qmax: uroflowmetry (UFR) measure of maximum flow rate PVR: postvoid residual volume | | |

While preferred embodiments of the present invention have been shown and described herein, it will be obvious to those skilled in the art that such embodiments are provided by way of example only.

The following preferred embodiments are also described:
1. A composition for treating benign prostatic hyperplasia (BPH) in a subject comprising an extract or powder of a herbal mixture comprising *Astragalus membranaceus* and *Glycine max,* and an anti-BPH agent.
2. The composition of claim 1, wherein said extract or powder of *Astragalus membranaceus* comprises at least one, two, three, or four components selected from the group consisting of polysaccharides, triterpenoid saponins, flavonoids, and amino acids.
3. The composition of claim 2, wherein said polysaccharides comprise alfalfa polysaccharide-1 (APS-1), alfalfa polysaccharide-1(APS-2), or combinations thereof.
4. The composition of claim 2, wherein said triterpenoid saponins comprise acetylastragaloside I, astragaloside I, astragaloside II, astragaloside IV, astramembrannin II, or combinations thereof.
5. The composition of claim 2, wherein said extract or powder of *Glycine max* comprises Genistein, Daidzein, or combination thereof.
6. The composition of claim 1, wherein said extract or powder of *Astragalus membranaceus* and *Glycine max* is in a ratio of 1:2 to 24:1 by weight.
7. The composition of claim 1, wherein the anti-BPH agent is an alpha blocker, a 5α-reductase inhibitor, or a combination thereof.
8. The composition of claim 7, wherein said alpha blocker is doxazosin, terazosin, alfuzosin, phentolamine, phenoxybenzamine, prazosin, tamsulosin, and silodosin, or its pharmaceutically acceptable salt, or solvate thereof.
9. The composition of claim 7, wherein said 5α-reductase inhibitor is dutasteride, epristeride, finasteride, izonsteride, turosteride, Ganoderic acid A, AS-601811, FK143, TF-505, or its pharmaceutically acceptable salt, or solvate thereof.
10. The composition of claim 1, wherein the anti-BPH agent is selected from the group consisting of doxazosin, terazosin, alfuzosin, tamsulosin, silodosin, finasteride, dutasteride, phenoxybenzamine, prazosin, tolterodine, sildenafil citrate, tadalafil, dutasteride, epristeride, finasteride, izonsteride, turosteride, Ganoderic acid A, AS-601811, FK143, TF-505, their pharmaceutical salts, or solvates thereof, and combinations thereof.
11. The composition of claim 1, where said composition reduces prostate volume or the severity of BPH based on International Prostate Symptom Score (IPSS) or Quality of Life (QoL) index.
12. The composition of claim 1, wherein said composition improves urine flow rate or nocturia of the subject.
13. A method for treating benign prostatic hyperplasia (BPH) in a subject comprising a step of administering an extract or powder of a herbal mixture as an add-on therapy to said subject wherein said herbal mixture comprises *Astragalus membranaceus* and *Glycine max.*
14. The method of claim 13, wherein said extract or powder of *Astragalus membranaceus* comprises at least one, two, three, or four components selected from the group consisting of polysaccharides, triterpenoid saponins, flavonoids, and amino acids.
15. The method of claim 14, wherein said subject is administering an anti- BPH agent with said add-on therapy.

## Claims

1. A composition suitable for use in treating benign prostatic hyperplasia (BPH) in a subject comprising an extract or powder of a herbal mixture comprising *Astragalus membranaceus* and *Glycine max,* and an anti-BPH agent, wherein said anti-BPH agent is tamsulosin or doxazosin, or its pharmaceutical salt, or solvate thereof.

2. The composition of claim 1, wherein said extract or powder of *Astragalus membranaceus* comprises at least one, two, three, or four components selected from the group consisting of polysaccharides, triterpenoid saponins, flavonoids, and amino acids.

3. The composition of claim 2, wherein said polysaccharides comprise alfalfa polysaccharide-1 (APS-1), alfalfa polysaccharide-1(APS-2), or combinations thereof.

4. The composition of claim 2, wherein said triterpenoid saponins comprise acetylastragaloside I, astragaloside I, astragaloside II, astragaloside IV, astramembrannin II, or combinations thereof.

5. The composition of claim 2, wherein said extract or powder of *Glycine max* comprises Genistein, Daidzein, or combination thereof.

6. The composition of claim 1, wherein said extract or powder of *Astragalus membranaceus* and *Glycine max* is in a ratio of 1:2 to 24:1 by weight.

7. The composition of claim 1, where said composition reduces prostate volume or the severity of BPH based on International Prostate Symptom Score (IPSS) or Quality of Life (QoL) index.

8. The composition of claim 1, wherein said composition improves urine flow rate or nocturia of the subject.

9. A composition for use in the treatment of benign prostatic hyperplasia (BPH) in a subject comprising an extract or powder of a herbal mixture as an add-on therapy to said subject taking tamsulosin or doxazosin, or its pharmaceutical salt, or solvate thereof, wherein said herbal mixture comprises *Astragalus membranaceus* and *Glycine max.*

10. The composition of claim 9, wherein said extract or powder of *Astragalus membranaceus* comprises at least one, two, three, or four components selected from the group consisting of polysaccharides, triterpenoid saponins, flavonoids, and amino acids.

## Patentansprüche

1. Zusammensetzung, die zur Verwendung beim Behandeln von benigner Prostatahyperplasie (BPH) in einem Subjekt geeignet ist, umfassend einen Extrakt oder ein Pulver einer Pflanzenmischung, die *Astragalus membranaceus* und *Glycine max* umfasst, und ein Anti-BPH-Mittel, wobei das Anti-BPH-Mittel Tamsulosin oder Doxazosin oder sein pharmazeutisches Salz oder Solvat davon ist.

2. Zusammensetzung nach Anspruch 1, wobei das Extrakt oder Pulver von *Astragalus membranaceus* mindestens einen, zwei, drei oder vier Bestandteile umfasst, die aus der Gruppe bestehend aus Polysacchariden, Triterpenoidsaponinen, Flavonoiden und Aminosäuren ausgewählt sind.

3. Zusammensetzung nach Anspruch 2, wobei die Polysaccharide Alfalfa-Polysaccarid-1 (APS-1), Alfalfa-Polysaccarid-1 (APS-2) oder Kombinationen davon umfassen.

4. Zusammensetzung nach Anspruch 2, wobei die Triterpenoidsaponine Acetylastragalosid I, Astragalosid I, Astragalosid II, Astragalosid IV, Astramembranin II oder Kombinationen davon umfassen.

5. Zusammensetzung nach Anspruch 2, wobei der Extrakt oder das Pulver von *Glycine max* Genistein, Daidzein oder Kombinationen davon umfasst.

6. Zusammensetzung nach Anspruch 1, wobei der Extrakt oder das Pulver von *Astragalus membranaceus* und *Glycine max* in einem Verhältnis von 1:2 bis 24:1 bezogen auf das Gewicht ist.

7. Zusammensetzung nach Anspruch 1, wobei die Zusammensetzung das Prostatavolumen oder die Schwere der BPH basierend auf dem International Prostata Symptom Score (IPSS) oder dem Quality of Life (QoL)-Index verringert.

8. Zusammensetzung nach Anspruch 1, wobei die Zusammensetzung die Harnflussrate oder Nykturie des Subjekts verbessert.

9. Zusammensetzung zur Verwendung in der Behandlung von benigner Prostatahyperplasie (BPH) in einem Subjekt, umfassend einen Extrakt oder ein Pulver einer Pflanzenmischung als adjuvante Therapie für das Subjekt zum Einnehmen von Tamsulosin oder Doxazosin oder seines pharmazeutischen Salzes oder Solvats davon, wobei die Pflanzenmischung *Astragalus membranaceus* und *Glycine max* umfasst.

10. Zusammensetzung nach Anspruch 9, wobei der Extrakt oder das Pulver von *Astragalus membranaceus* mindestens einen, zwei, drei oder vier Bestandteile umfasst, die aus der Gruppe bestehend aus Polysacchariden, Triterpenoidsaponinen, Flavonoiden und Aminosäuren ausgewählt sind.

## Revendications

1. Composition appropriée pour une utilisation dans le traitement de l'hyperplasie bénigne de la prostate (HBP) chez un sujet comprenant un extrait ou une poudre d'un mélange à base de plantes comprenant de l'*Astragalus membranaceus* et de la *Glycine max,* et un agent anti-HBP, ledit agent anti-HBP étant la tamsulosine ou la doxazosine, ou son sel pharmaceutique, ou un solvate de celui-ci.

2. Composition selon la revendication 1, dans laquelle ledit extrait ou ladite poudre d'*Astragalus membranaceus* comprend au moins un, deux, trois ou quatre composants choisis dans le groupe constitué par les polysaccharides, les saponines triterpénoïdes, les flavonoïdes et les acides aminés.

3. Composition selon la revendication 2, dans laquelle lesdits polysaccharides comprennent le polysaccharide-1 de la luzerne (APS-1), le polysaccharide-2 de la luzerne (APS-2), ou des combinaisons de ceux-ci.

4. Composition selon la revendication 2, dans laquelle lesdites saponines triterpénoïdes comprennent l'acétylastragaloside I, l'astragaloside I, l'astragaloside II, l'astragaloside IV, l'astramembranine II ou des combinaisons de ceux-ci.

5. Composition selon la revendication 2, dans laquelle ledit extrait ou ladite poudre de *Glycine max* comprend de la génistéine, de la daidzéine ou une combinaison de celles-ci.

6. Composition selon la revendication 1, dans laquelle ledit extrait ou ladite poudre d'*Astragalus membranaceus* et de *Glycine max* est dans un rapport pondéral de 1:2 à 24:1.

7. Composition selon la revendication 1, dans laquelle ladite composition réduit le volume de la prostate ou la gravité de l'HBP sur la base du score international des symptômes de la prostate (IPSS pour « International Prostate Symptom Score ») ou de l'indice de la qualité de vie (QoL).

8. Composition selon la revendication 1, dans laquelle ladite composition améliore le débit urinaire ou la nycturie du sujet.

9. Composition pour une utilisation dans le traitement de l'hyperplasie bénigne de la prostate (HBP) chez un sujet comprenant un extrait ou une poudre d'un mélange à base de plantes en tant que thérapie d'appoint audit sujet prenant de la tamsulosine ou de la doxazosine, ou son sel pharmaceutique, ou un solvate de celui-ci, ledit mélange à base de plantes comprenant de l'*Astragalus membranaceus* et de la *Glycine max.*

10. Composition selon la revendication 9, dans laquelle ledit extrait ou ladite poudre d'*Astragalus membranaceus* comprend au moins un, deux, trois ou quatre composants choisis dans le groupe constitué par les polysaccharides, les saponines triterpénoïdes, les flavonoïdes et les acides aminés.
